# EUROPEAN PATENT APPLICATION

(11) **EP 2 402 712 A1**
(43) Date of publication of application: **04.01.2012**
(21) Application number: 11005189.3
(22) Date of filing: 27.06.2011
(51) Int. Cl.: G01B 11/24, A61C 13/00, G01P 3/36

(54) **Method for the digital survey of at least one body by a laser sensor and related device**

(30) Priority: 28.06.2010 IT PG20100040
(71) Applicant: Iride S.r.l., 06154 Perugia (IT)
(72) Inventor: Logozzo, Silvia, 06129 Perugia (PG) (IT); Caponi, Michele, 06154 Perugia (PG) (IT); Franceschini, Giordano, 06125 Perugia (PG) (IT)
(74) Representative: Marietti, Andrea

(57) **Abstract**

Method for digitally surveying at least one body (100), by a laser sensor (1), to acquire at least one tridimensional cloud (N) of points representative of at least part of said at least one body, wherein the position of said laser sensor (1) and every said points of said at least one cloud of points is referred, directly or indirectly, to a fixed reference frame (x,y,z); said method comprising the steps of: a) mutually moving said laser sensor (1) relatively to said body (100); b) acquiring one or more profiles of points P(T0), P(T1), P(T2), P(Ti)...P(Tf) associated with corresponding instants (Ti) of at least one plurality of following acquisition instants (T0, T1, T2, Ti..., Tn), to form said at least one cloud of points (N) representative of at least part of said at least one body (100); c) establishing the velocity (Vcorp) of said at least one body for each instant of said at least one plurality of following acquisition instants (T0, T1, T2, Ti..., Tf); and d) interrupting said step b) of the method based on the value of velocity (Vcorp) of said at least one body (100) determined during said step c) of the method.

## Description

The present invention concerns a method for the digital survey of a body by a laser scanner sensor and related device.

In particular, such a method uses a 3D laser scanner sensor to acquire the three-dimensional geometry of a body through the survey in the space of a plurality of representative points of the surface shape and size of the body itself. In particular, such a method and related device are suitable to precisely survey the geometry of a body that can move relatively to the laser scanner sensor, moving too, during the scan. More particularly, such a method and related device can be advantageously used in the field of dental prostheses for the acquisition of the dentition shape of a patient in vivo.

According to the known art, the 3D laser scanner sensors moved manually by an operator, are normally used in the survey of the geometry of a completely still body, such as a mechanical component, an archaeological find or the like. Initially, the sensor acquires, in the surrounding space and with a certain defined resolution, a plurality of profiles of representative points on the whole of the body to be surveyed. Subsequently, through the use of well-known graphics processing methods, the points of all acquired profiles are interpolated one another, for example with the use of NURBS or triangulation methods, to obtain the numerical equation of the surveyed surface. In this case, programs ordinary in the drawing to computer field are also used, allowing to obtain the digital surface and, in case, the digital volume too of the body to be surveyed. It has to be noted that the profiles of acquired points, depending on the used sensor, may be either simple lines, having a development on a plane parallel to the sensor itself, or real surfaces, i.e. having a dimension also in the cross direction relatively the sensor itself.

Also according to the known art, in the case of very large surfaces, 3D laser scanner sensors can be displaced with a certain velocity relatively to the body to surveyed, so that the entire surface to be surveyed could be acquired in a certain amount of time.

If the sensor is displaced relatively to a completely still body, it is necessary that the sensor position with respect to a fixed reference frame is always known during the movement of the sensor itself. However, in traditional acquisition systems is always necessary that the body under scansion is perfectly still because it is not possible to identify the instant when a determined profile of points of said cloud of points was obtained. The entire acquisition process is therefore to be completely stationary, i.e. time - invariant.

By using these sensors, possible and imperceptible movements of the body to surveyed can irreparably compromise the entire carried out measurement. This therefore makes impossible to use the known 3D laser sensors for the survey of bodies whose movements relatively to the same sensor occur intermittently, randomly, and however, not uniformly in space and time. For example, it is not possible to use known 3D laser scanner sensors for the acquisition of a patient dentition because the movements of the patient mandible and / or the head itself of the latter during the acquisition step, would not make reliable the carried out measurements. In practice, during the step of processing the acquired information, commonly known as post-processing, it would be difficult to assess at what time the error occurred, however obtaining a surface that is composed of points that, acquired in different moments, however refer to the same portion of the body to be surveyed, at least in part. In conclusion, the final surface is composed of redundant profiles, i.e. overlapped, creating a highly undesirable trail effect. The object of the present invention is to realize a method for digitally surveying a body surface by using 3D laser scanner sensor allowing to survey the outer surface of a body with very little measurement errors, preferably at least a dentition portion of a patient, also in case wherein such a body is not absolutely still, but it is randomly and unpredictably moving relatively to the said 3D laser sensor.

A further object of the present invention is to realize a 3D laser sensor that is of simple construction and easily handled by the operator.

These and other objects are obtained by the present method for digitally surveying at least one body, by a laser sensor, to acquire at least one tridimensional cloud of points representative of said at least one body, wherein the position of said laser sensor and every said points of said cloud of points is referred, directly or indirectly, to a fixed reference frame; said method comprising the steps of: a) mutually moving said laser sensor relatively to said body; b) acquiring one or more point profile associated with corresponding instants of at least one plurality of following acquisition instants, to form said at least one cloud of points representative of at least part of said at least one body; c) establishing the velocity of said at least one body for each instant of said at least one plurality of following acquisition instants; and d) interrupting said step b) of the method based on the value of velocity of said at least one body.

In this way, according to the invention, in the immediate following, it is possible to acquire multiple profiles of points to form at least a portion of the cloud of points representative of said body to be surveyed. Such an acquisition may continue until the body to be surveyed is not still, or it has a very low velocity. In the moment wherein a velocity of said body above a certain preset value is determined, e.g. 0.05 m/s, the acquisition is stopped to avoid problems of acquisition trails encountered in the 3D laser scanner sensor of the known art. Further, the method comprises the further step of e) cyclically repeating each step from a) to d) to obtain a plurality of clouds of points each representative of at least part of said at least one body. In practice, the acquisition is stopped and it is resumed in order to acquire one or more clouds of points only when the body, that it is needed to be digitally surveyed, is essentially still.

According to a particular aspect of the invention, said step c) of the method comprises the steps of: f) determining for each instant of said plurality of acquisition instants the velocity of said laser sensor; g) determining for each instant of said plurality of acquisition instants the relative velocity between said at least one body and said at least one sensor; h) for each instant of said plurality of acquisition instants, comparing said relative velocity and said velocity of said at least one sensor, to determine said velocity of said at least one body.

In particular, the velocity of said sensor is calculated using the inverse of the ratio between the time interval from an acquisition instant and the subsequent and the device displacement between said two instants. Such a displacement is easily determined as the coordinates of the sensor relatively to the afore said fixed reference are always known.

Further, the method comprises the step of i) reconstructing by mathematic processes a tridimensional solid model of said at least one body starting from said one or more clouds of points acquired during said steps of the method from a) to d) and / or e).

Said step i) comprises, as well, the step of I) triangulating, or m) mathematically interpolating one to each other the points of each cloud of points acquired during said steps of the method from a) to d) and / or e) using the NURBS functions, to obtain a digital outer surface of each of said one or more clouds of points. Afterwards, the said step I), or the said step m), is followed by the step of n) overlapping one to each other said outer digital surfaces of said one or more clouds of points, calculated during said step I), or said step m), of the method, to continuously align one to each other the outer digital surfaces having surface portions geometrically mutually coincident, to obtain an outer digital surface of said at least one body.

It has to be noticed that for geometrically coincident it is intended that the two surfaces are morphologically identical, i.e. they have an identical shape, they are dimensionally equivalent and spatially aligned.

In conclusion, the 3D laser sensor is displaced with respect to the dentition portion of the patient to acquire, at any time, profiles of points until a patient displacement is surveyed. In this case, the acquisition is stopped however generating a first cloud of points representing at least a portion of the body to survey, specifically the dentition of the patient. Then such an acquisition may be restarted either from the point where it has been stopped, or even before it, until the end of the dentition portion to be surveyed or until the patient does not move again. Then, thanks to the interpolation, or mathematical triangulation, the single clouds of points consisting of acquired profile points are transformed into distinct surfaces. At this point the single surfaces are overlapped with each other and joined together continuously only if they are provided with portions geometrically coincident with each other.

Finally, the said step i) of the method is followed by step of o) obtaining a prototype of said at least one body by rapid prototypization starting from said solid model obtained when the outer digital surface of said at least one body is known.

A further object of the present invention is to realized a device for digitally surveying at least one body, comprising a laser sensor to acquire at least one tridimensional cloud of points representative of said at least one body, during at least one plurality of following acquisition instants, wherein the position of said laser sensor and said points of said cloud of points is referred, directly or indirectly, to a fixed reference frame; said device further comprises means for determining a velocity of said at least one body, means for acquiring at least one point profile of said at least one cloud of points of said body, associated with an instant of said at least one plurality of acquisition instants, and means for interrupting said acquisition based on the value of velocity of said body determined by said means for determining the velocity of said at least one body. Further, said means for determining the velocity of said at least one body are integral with said at least one laser sensor and comprise as well at least one Doppler effect sensor to determine the relative velocity between said at least one body and said laser sensor, at least one logic unit to determine the velocity of said at least one laser sensor and at least one comparator to compare said velocity of said at least one laser sensor with the velocity of said at least one body.

For purposes of illustrations and not limitative, a particular embodiment of the present invention will be described with reference to the accompanying figures, in which:
figure 1 is a perspective view of a plurality of portion profiles of human dentition acquired in different instants;
figure 2a is a perspective view of one or more clouds of points acquired during the steps from a) to e) of the method;
figure 2b is a perspective view of multiple digital surfaces obtained during the step I) or m) of the method;
figure 3 is a view of the operating scheme of the device according to the invention.

Particularly referring to such figures, a device 200 is indicated for digitally surveying at least one body according to the invention.

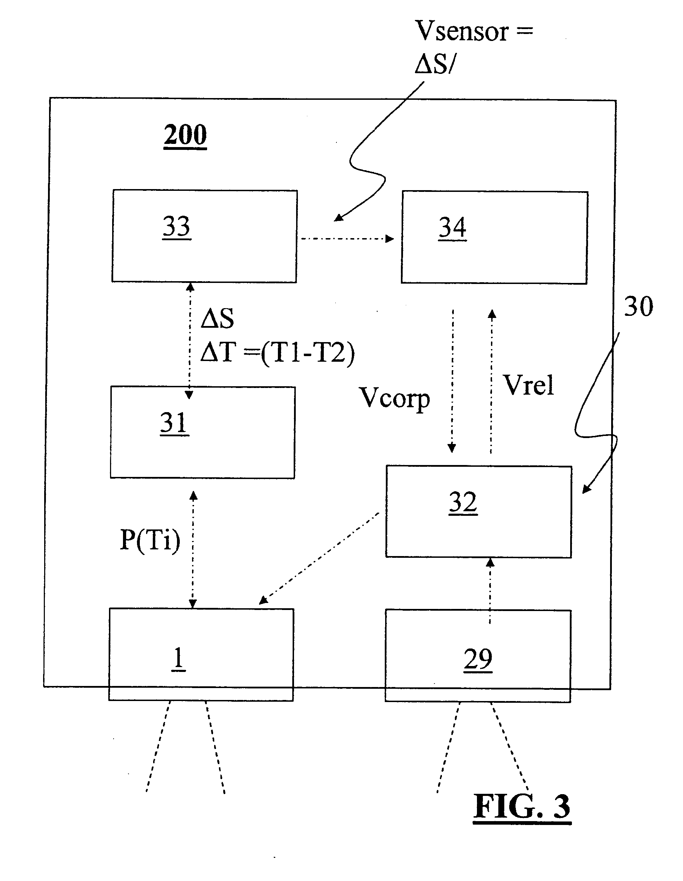

The device 200 (see Figure 3) comprises a laser sensor 1 for the acquisition of tridimensional N clouds of points representative of a body 100, specifically the dentition of a patient, during an acquisition process occurring during at least a plurality of following acquisition instants T0, T1, T2, Ti ...., Tn. The position of said laser sensor 2 and said points of said cloud of points is indirectly related to a fixed reference frame x, y, z with respect to which it is possible to know, by the use of common transformation matrices of the position of points, the spatial position of both the laser sensor 1 and the points acquired by the sensor. Advantageously such a laser sensor 1 comprises means 30 for determining the velocity Vcorp of said body 100, means 31 for acquiring more profiles of points P(Ti) of at least one cloud of points N of said body 1, wherein each profile is associated with an instant Ti of said plurality of acquisition instants T0, T1, T2, Ti ...., Tn, and means 32 for interrupting said acquisition based on the value of velocity of said body determined by said means 30 for determining the velocity Vcorp of said body 100. In such a way it is possible to generate a plurality of profiles P(T0), P(T1), P(T2), P(Ti) ... P(Tn) of points at following distinct instants T0, T1, T2, ...., Tn, representing at least part of said body 100.

The afore said means 30 for determining the velocity Vcorp of said at least one body are integral with said at least one laser sensor 1 and comprise as well at least one Doppler effect sensor 29 to determine the relative velocity Vrel between said at least one body and said laser sensor 1, at least one logic unit 33 to determine the velocity Vsensor of said laser sensor 1 and at least one comparator 34 to compare said velocity Vsensor of said at least one laser sensor with said relative velocity Vrel. In practice, the difference between these two velocities, respectively, the velocity Vsensor of the laser sensor 1 and the relative velocity Vrel, causes the determination of the velocity Vcorp of the body 100. The above mentioned logic unit 33 calculates the velocity Vsensor of the laser sensor 1 simply carrying out the ratio between the displacement AS performed by the sensor 1 during two following acquisition instants T1-T2, wherein the interval AT coincides with the acquisition period of the sensor 1, and such an interval. At any moment it is therefore possible to know the velocity of the laser sensor 1 because it is always possible to determine the displacement AS of the laser sensor 1. This is due to the fact that the acquired profiles are referred to a determined instant and, therefore, it is always possible to calculate the velocity of the sensor because the movements in the space are known.

It has to be noticed that, although it has been referred afore to a Doppler effect sensor 29 as a means to determine the relative velocity Vrel between said sensor and said body, however, any device able to measure the relative velocity between two moving elements in real time still falls in the protection scope of the present invention. For example, a sonar or radar may be used for such an object.

According to the method object of the present invention, the digital survey of the afore said body 100, preferably a portion of human dentition, is achieved by a laser sensor 1 for the acquisition of at least one tridimensional cloud N of points representing said at least one body 100. It has to be noticed that the position of said laser sensor 1 and each of said points of said at least one cloud N of points is referred, directly or indirectly, to a fixed reference frame x, y, z, and, therefore, at any time it is possible to know the absolute position of the sensor 1, its movements and hence its velocity. It has to be noticed, in fact, that, as mentioned above, and implicitly intended in the step b) of the method (as will be evident herein below), between two acquisitions of following profiles the elapsed interval AT is always the same, the latter coinciding with the sampling, or acquisition, interval of the laser scanner 1. As a result of what mentioned, it is always possible to known the absolute position of the scanner between an instant and another and, hence, the velocity of the scanner itself.

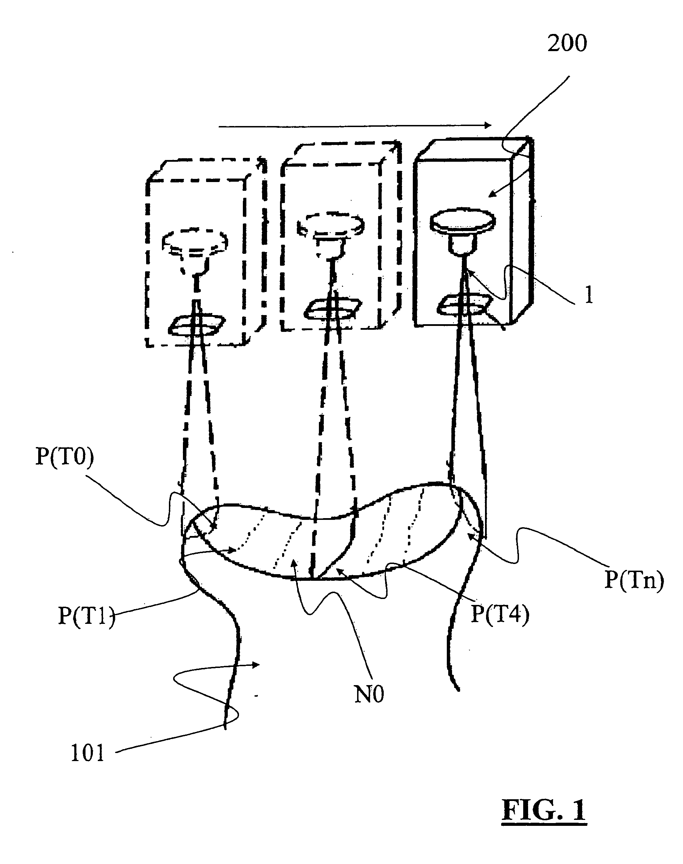

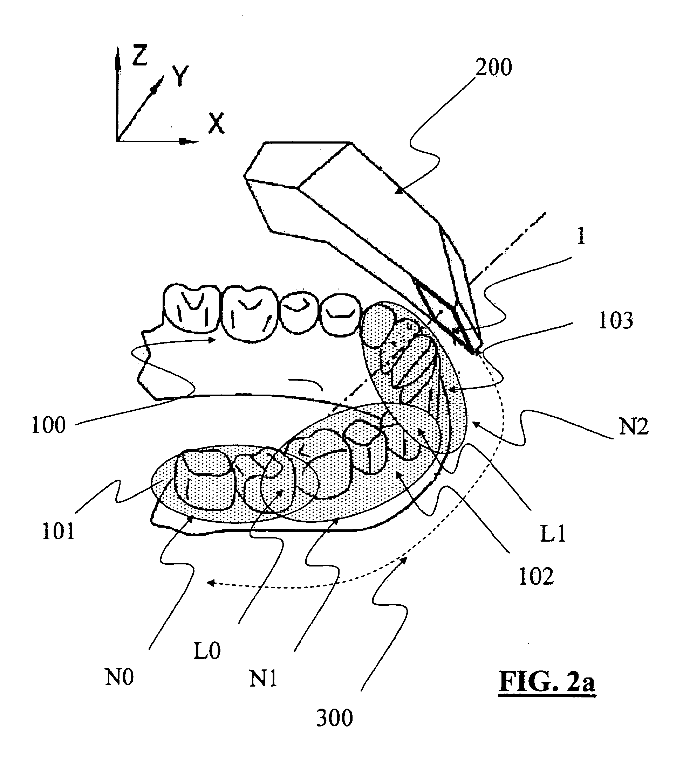

The method comprises, however, the steps of: a) reciprocally displacing said laser sensor 1 relatively to said dentition 100 (see Figures 1 and 2a), b) acquiring a plurality of profiles P(T0), P(T1), P(T2), P(Ti) ... P(Tf) of points associated with corresponding instants of said plurality of following acquisition instants T0, T1, T2 ,...., Tn, to form at least a cloud N0 of points representative of at least part of said dentition 100 (see Figure 1), c) determining for each instant Ti of said plurality following acquisition instant T0, T1, T2 ,...., Tn the velocity Vcorp of said at least one body 100; and d) interrupting said step b) of the method based on the value of velocity Vcorp of said at least one body. Preferably, said value of velocity Vcorp of said body 100 is between 0 and 0.05 m/s. Basically such a velocity value must be zero.

In this way, according to the invention, it is possible to acquire multiple profiles of points in following instants to form a cloud of points representative of at least part of the body to be surveyed. Such an acquisition may continue until the body 100 to be surveyed is not still, or it has a very low velocity. In the moment wherein a velocity of said body above a certain preset value is determined, e.g. 0.05 m/s, the acquisition is stopped to avoid problems of acquisition trails encountered in the 3D laser scanner sensor of the known art.

Also according to the invention, the method comprises the step of e) cyclically repeating the step from a) to d) to obtain, as shown in Figure 2a, two other clouds N1 and N2 of points, each of which is representative of two other portions 102, 103 of said dentition 100. In conclusion, according to the example herein shown, the sensor 1 has been displaced in relation to said dentition 100 (see arrow 300) in at least three occasions, the clouds N0, N1, N2 being acquired in number of three, and the velocity Vcorp is higher for as many times than a certain value with the consequent shutdown of the acquisition.

More in detail said step c) of the method comprises the steps of: f) determining for each instant Ti of said plurality of following acquisition instants T0,T1,T2,Ti,....,Tn the velocity Vsensor of said laser sensor 1; g) determining for each instant Ti of said plurality of following acquisition instants T0,T1,T2,Ti,....,Tn the relative velocity Vrel between said dentition 100 and said laser sensor 1; h) for each instant Ti of said at least one plurality of acquisition instants T0,T1,T2,Ti,....,Tn, comparing said relative velocity Vrel with said velocity Vsensor of said at least one sensor, to determine said velocity Vcorp of said dentition 100. The method thus allows to detect the movement of the body 100 to be surveyed by a direct measure of the relative velocity between said dentition 100 and an indirect measure of the velocity Vsensor of said sensor 1. Such a velocity Vsensor of said sensor 1 is calculated by the ratio of the displacement AS of the sensor 1 in two following acquisition instants, for example, T1-T2, and the time interval ΔS between said two instants T1-T2. Substantially, said time interval AS being constant, this coinciding with the acquisition interval of the sensor, the only value that is calculated is the value of the displacement ΔS made by the sensor. Such a displacement, however, can be obtained simply because it is always possible to know the spatial position of the sensor 1 with respect to a fixed reference x, y, z.

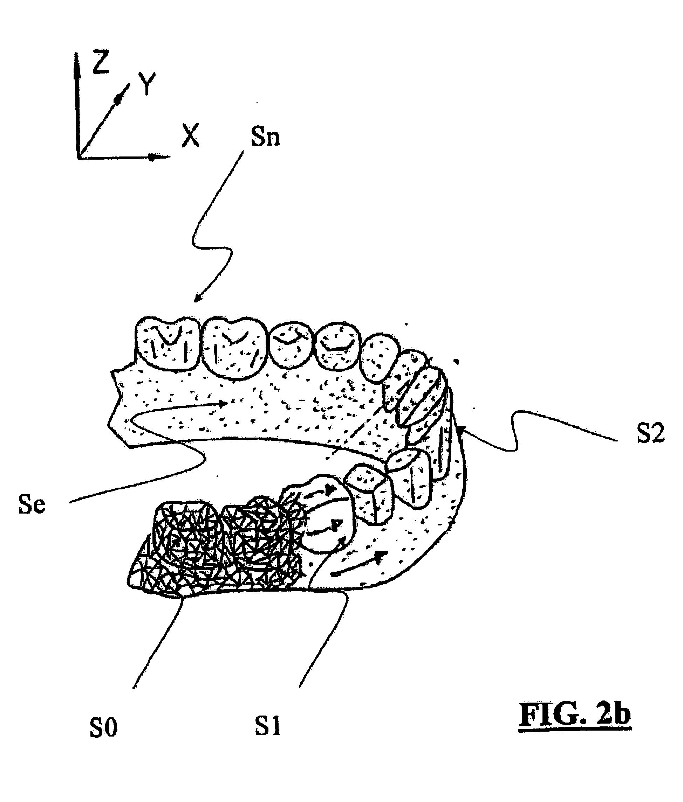

After said step e), the method comprises as well the step of i) reconstructing by mathematic processes a tridimensional solid model M of said dentition 100 starting from said three clouds of points N0, N1, N2 acquired during said steps of the method from a) to e). Said step i) comprises as well the step of l) triangulating, or alternatively (here not shown) of m) mathematically interpolating one to each other the points of each of the three clouds N0, N1, N2 of points acquired during said steps of the method from a) to e) using the NURBS functions, to obtain a digital outer surface S0, S1, S2 of each of said three clouds of points N0, N1, N2 (for easiness in figure 2b only the surface S0 is shown). Afterwards, said step l), or said step m), is followed by the step of n) overlapping one to each other said outer digital surfaces S0, S1, S2 of said clouds of points S0, S1, S2, calculated during said step l), or said step m), of the method, to continuously align one to each other the outer digital surfaces S0, S1, S2 having surface portions L0, L1 geometrically mutually coincident, to obtain an outer digital surface Se of said dentition (see figure 2a wherein the overlapped surfaces L0, L1 are schematically shown). It has to be observed that although the above reported description has three clouds, however, a number of clouds of points exceeding three or below such a number, for example, a single cloud, yet falls within the protection scope of the present invention. It has also to be noticed that the number of geometrically coincident surface portions may be different from two without for this reason leaving the protection scope of the present invention.

Finally, the method of said step i) of the method is followed by step of o) obtaining a prototype of said dentition by rapid prototypization starting from said solid model M. In conclusion, thanks to such a step, it is possible to obtain a mold of the dentition of the patient for the possible generation of dental prostheses.

## Claims

1. Method for digitally surveying at least one body (100), by a laser sensor (1), to acquire at least one tridimensional cloud (N) of points representative of at least part of said at least one body, wherein the position of said laser sensor (1) and every said points of said at least one cloud of points is referred, directly or indirectly, to a fixed reference frame (x,y,z); said method comprising the steps of:
a) mutually moving said laser sensor (1) relatively to said body (100);
b) acquiring one or more profiles of points P(T0), P(T1), P(T2), P(Ti)...P(Tf) associated with corresponding instants (Ti) of at least one plurality of following acquisition instants (T0, T1, T2, Ti..., Tn), to form said at least one cloud of points (N) representative of at least part of said at least one body (100);
c) establishing the velocity (Vcorp) of said at least one body for each instant of said at least one plurality of following acquisition instants (T0, T1, T2, Ti..., Tf); and
d) interrupting said step b) of the method based on the value of velocity (Vcorp) of said at least one body (100) determined during said step c) of the method.

2. Method according to claim 1, **characterized by** comprising the step of e) cyclically repeating said steps from a) to d) to obtain a plurality of clouds of points (N, N0, N1, N2, ..., Nn), each of them being representative of at least part of said at least one body (100).

3. Method according to one or more of the preceding claims, **characterized in that** said velocity of said at least one body is comprised between 0 and 0.05 m/s.

4. Method according to one or more of the claims from 1 to 3, **characterized in that** said step c) of the method comprises the steps of: f) determining for each instant of said at least one plurality of following acquisition instants (T0, T1, T2, Ti..., Tf) the velocity (Vsensor) of said laser sensor (1); g) determining for each instant of said at least one plurality of following acquisition instants (T0, T1, T2, Ti..., Tf) the relative velocity (Vrel) between said at least one body (100) and said at least one sensor (1); h) for each instant of said at least one plurality of following acquisition instants (T0, T1, T2, Ti..., Tf), comparing said relative velocity (Vrel) and said velocity (Vsensor) of said at least one sensor, to determine said velocity (Vcorp) of said at least one body (100).

5. Method according to claim 4, **characterized in that** said sensor velocity (Vsensor) is calculated by the ratio between the displacement (ΔS) of said sensor between two following acquisition instants (T0-T1, T1-T2, T2-T3....Tf-1-Tf) and the time interval comprised between said two following acquisition instants (ΔT).

6. Method according to one or more of the preceding claims, **characterized by** comprising the step of i) reconstructing by mathematic processes a tridimensional solid model (M) of said at least one body (100) starting from said one or more clouds of points (N, N0, N1, N2,...,Nn) acquired during said steps of the method from a) to d) and / or e).

7. Method according to claim 6, **characterized in that** said step i) comprises the step of I) triangulating, or m) mathematically interpolating one to each other the points of each cloud (N0, N1, N2,...,Nn) acquired during said steps of the method from a) to d) and / or e) using the NURBS functions, to obtain a digital outer surface (S0, S1, S2,.., Sn) of each of said one or more clouds of points (N0, N1, N2,...,Nn).

8. Method according to claim 7, **characterized in that** said step I), or said step m), is followed by the step of n) overlapping one to each other said outer digital surfaces (S0, S1, S2,.., Sn) of each cloud of said one or more clouds of points (N0, N1, N2,...,Nn) acquired during said steps of the method from a) to d) and / or e), to continuously align one to each other said outer digital surfaces (S0, S1, S2,..., Sn) having surface portions (L0, L1, L2,...,Ln) geometrically mutually coincident, to obtain an outer digital surface (Se) of said at least one body (100).

9. Method according one or more of the claims from 6 to 8, **characterized in that** said step i) of the method is followed by the step of o) obtaining a prototype of said at least one body (100) by rapid prototypization starting from said solid model (M).

10. Method according to one or more of the preceding claims, **characterized in that** said at least one body (100) comprises at least one dentition portion of a patient.

11. Device (200) for digitally surveying at least one body (100), comprising a laser sensor (2) to acquire at least one tridimensional cloud (N) of points representative of said at least one body (1), during at least one plurality of following acquisition instants (T0, T1, T2, Ti....,Tf), wherein the position of said laser sensor (2) and every said points of said cloud of points is referred, directly or indirectly, to a fixed reference frame (x,y,z), said device (200) for digitally surveying comprising means (29) for determining a velocity of said at least one body (100), means (31) for acquiring one or more profiles of points (P(Ti)) of one or more clouds of points (N, N0, N1, N2,...,Nn) of said at least one body (100), wherein each profile is associated with an instant (Ti) of said at least one plurality of acquisition instants (T0, T1, T2, Ti....,Tn), and means (32) for interrupting said acquisition based on the value of velocity (Vcorp) of said at least one body (100), determined by said means (30) for determining the velocity of said at least one body.

12. Device according to claim 11, **characterized in that** said means (29) for determining the velocity (Vcorp) of said at least one body (100) are integral with said at least one laser sensor (1) and comprise as well at least one Doppler effect sensor (29) to determine the relative velocity (Vrel) between said at least one body (100) and said laser sensor (1), at least one logic unit (33) to determine the velocity of said at least one laser sensor (1) and at least one comparator (34) to compare said velocity (Vsensor) of said at least one laser sensor with the velocity (Vcorp) of said at least one body (100).
